# EUROPEAN PATENT SPECIFICATION

(11) **EP 1 159 882 B1**
(45) Date of publication and mention of the grant of the patent: **05.04.2006**
(21) Application number: 01401409.6
(22) Date of filing: 30.05.2001
(51) Int. Cl.: A23L 1/22, A23P 1/04

(54) **Coating material and coated powder**
Beschichtungsmaterial und beschichtetes Pulver
Matériau d'enrobage et poudre enrobée

(30) Priority: 30.05.2000 JP 2000159675; 28.03.2001 JP 2001092589
(43) Date of publication of application: 05.12.2001
(73) Proprietor: Takasago International Corporation, Tokyo (JP)
(72) Inventor: Hirai, Nobuhisa, c/o Takasago International Corp., Hiratsuka-shi, Kanagawa (JP); Nagao, Masaharu, c/o Takasago International Corp., Hiratsuka-shi, Kanagawa (JP); Uno, Katuya, c/o Takasago International Corp., Hiratsuka-shi, Kanagawa (JP); Ishii, Hiroshi, c/o Takasago International Corp., Hiratsuka-shi, Kanagawa (JP)
(74) Representative: Uchida, Kenji

(56) References cited:
- US-A- 5 015 480
- DATABASE WPI Section Ch, Week 200019 Derwent Publications Ltd., London, GB; Class A11, AN 2000-218214 XP002276168 & JP 2000 044878 A (KIRIN BREWERY KK), 15 February 2000 (2000-02-15) -& PATENT ABSTRACTS OF JAPAN vol. 2000, no. 05, 14 September 2000 (2000-09-14) & JP 2000 044878 A (KIRIN BREWERY), 15 February 2000 (2000-02-15)

## Description

### BACKGROUND OF THE INVENTION

### FIELD OF THE INVENTION:

The present invention relates to a coated powder which can favorably be used as a fragrance- or flavor-imparting or strengthening material, or a colorant or as a perfume-imparting agent for perfumy cosmetics, and which shows a lower hygroscopicity, an excellent oxidation stability, and realizes lasting and controlled flavor release; a coating agent for use in producing the coated powder; a food or beverage; a perfumy cosmetic; and a process for producing the coated powder.

### BACKGROUND ART:

Various substances have heretofore been added to various foods, beverages, perfumy cosmetics for enhancing their tastes, imparting or strengthening fragrance or flavor, coloring them, strengthening their functions, or improving handling ease. In addition, conversion of the form of additives to powder has also been popularly conducted for the additives to effectively exhibit their performance in foods, beverages or perfumy cosmetics or for improving handling properties of the additives.

As methods of converting the form of these various additives to powder, methods of coating a powdery or granular material with a coating material, and coating materials for the coating, various techniques are known. For example, in forming a perfume, a spice oil, a spice oleoresin or a composition thereof into powder, it has widely been conducted to emulsify them using a solution of natural gum such as gum arabic, protein such as gelatin or processed starch such as dextrin, and spray drying the emulsion. In using natural gum, protein or processed starch for preparing the powder, the resusltant powders show unfavorable hygroscopicity and have the defect that perfumy ingredients volatilize away, effective ingredients undergo change, or discoloration takes place.

Therefore, various proposals have been made for solving these defects. For example, there are illustrated methods of coating powdery flavor or powder-formed flavor with an animal or vegetable hardened oil, a synthetic fat and oil, or a resin (Japanese Patent Publication No. 12600/1970, Japanese Patent Laid-Open No. 92242/1974, Japanese Patent Laid-Open No. 313092/1995, Japanese Patent Laid-Open No. 152857/1992 and Japanese Patent Laid-Open No. 65850/1997) and a method of coating a core substance of a flavor oil-adsorbed dextrin with pullulan (Japanese Patent Laid-Open No. 47378/1996).

Recently, Japanese Patent Laid-Open No. 44878/2000 proposes to use as a coating material a fractionated yeast cell wall comprising a yeast cell residue obtained by removing soluble intracellular ingredients from enzyme-treated yeast cells, or an acid-treated yeast cell fraction composed of a residue obtained by treating with an acidic aqueous solution a yeast cell residue prepared by removing soluble intracellular ingredients from enzyme-treated yeast cells, and these fractionated yeast cell walls being commercially available. Although this publication describes to incorporate a plasticizer in the coating material, it does not disclose to use as a coating material a combination of these fractionated yeast cell walls and at least one member selected from the group consisting of viscous polysaccharides, oligosaccharides, hardened fats and oils, waxes, erythritol, mannitol, lactitol and starch hydrolyzates.

As is described above, various materials and methods have so far been proposed. However, coated powders obtained by these conventional methods have, to a greater or lesser degree, the problem that sufficient film is not formed around the core substance or that, even when a coating substance is used in combination, there is a limit as to heat resistance, oxidation stability, controlled-release properties or like functions. Thus, there have been eagerly desired a coated powder in which a core substance is sufficiently coated with a coating film showing excellently low hygroscopicity and excellent heat resistance and which shows excellent oxidation stability of core substance, resistance to volatilization of flavor ingredients of the core substance, resistance to discoloration, and controlled-release properties of the ingredients; and a coating material to be used for producing the coated powder having such excellent properties.

### SUMMARY OF THE INVENTION

The present invention provides a coated powder in which a core substance is sufficiently coated with a coating film showing excellently low hygroscopicity and excellent heat resistance and which shows an excellent oxidation resistance of the core substance, and a coating material to be used for producing the coated powder having such excellent properties.

The invention also provides a coated powder comprising a core substance containing therein a flavor which have, in addition to the above-described various properties, more improved resistance of volatilization of the flavor ingredient, resistance to discoloration and controlled-release properties of the ingredient, and a coating material to be used for producing the coated powder having such excellent properties. Further, it provides a coated powder which, when added to food, beverage or perfumy cosmetic and heat-treated, shows excellent flavor ingredient-keeping properties and which gives controlled release of flavor within the mouth upon uptake, can control the period of flavor retention, and shows excellent oxidation stability; and a coating material to be used for producing the coated powder having such excellent properties.

Still further, the invention provides food, beverage or perfumy cosmetic containing the coated powder.

Yet further, the invention provides a process for producing the coated powder.

As a result of intensive investigations to solve the above-described problems, the inventors have found that a coated powder is excellent in various properties such as flavor-keeping properties, flavor release-controlling ability, and oxidation stability, when a powdery or granular core substance is coated with a coating material comprising (A)(a1) a fractionated yeast cell wall comprising a yeast cell residue obtained by removing soluble intracellular ingredients from enzyme-treated yeast or (a2) an acid-treated yeast cell fraction comprising a residue obtained by treating with an acidic aqueous solution a yeast cell residue prepared by removing soluble intracellular ingredients from enzyme-treated yeast cells, and removing solubilized ingredients therefrom (in the invention, both (a1) and (a2) being referred to as "fractionated yeast cell wall") and (B) at least one member selected from the group consisting of viscous polysaccharides, oligosaccharides, hardened fats and oils, waxes, erythritol, mannitol, lactitol and starch hydrolyzates, thus having completed the invention based on the finding.

The present invention includes the following embodiments of invention.
(1) A coating material containing a fractionated yeast cell wall and at least one member selected from the group consisting of viscous polysaccharides, oligosaccharides, hardened fats and oils, waxes, sugar alcohols and starch hydrolyzates.
(2) The coating material as described in (1) described above, wherein the viscous polysaccharide is pullulan.
(3) The coating material as described in (1) described above, wherein the oligosaccharide is at least one member selected from the group consisting of trehalose, paratinose and raffinose.
(4) A coated powder composed of a core substance in a powdery or granular form of 30 to 3000 µm in an average particle size coated with 0.05 to 1.5 parts by weight of a coating material containing a fractionated yeast cell wall and at least one member selected from the group consisting of viscous polysaccharides, oligosaccharides, hardened fats and oils, waxes, erythritol, mannitol, lactitol per 1 part by weight of the core substance.
(5) The coated powder as described in (4) described above, wherein the viscous polysaccharide is pullulan.
(6) The coated powder as described in (4) described above, wherein the oligosaccharide is at least one member selected from the group consisting of trehalose, paratinose and raffinose.
(7) The coated powder as described in one of (4) to (6) described above, wherein the core substance is a flavor composition, a colour material, an acidity regulator, a seasoning, a sweetener, a spice, a vitamin, a functional material or a mixture of two or more of them.
(8) The coated powder as described in (7) described above, wherein the core substance is a flavor composition.
(9) A food or beverage which contains the coated powder described in one of (4) to (8) described above.
(10) A perfumy cosmetic which contains the coated powder described in one of (4) to (8) described above.
(11) A process for producing a coated powder, which comprises spraying a solution of a coating material containing a fractionated yeast cell wall and at least one member selected from the group consisting of viscous polysaccharides, oligosaccharides, hardened fats and oils, waxes, erythritol, mannitol, lactitol and starch hydrolyzates against a core substance in a powdery or granular form of 30 to 3000 µm in an average particle size under stirring or in a fluidized state to thereby coat the core substance with 0.05 to 1.5 parts by weight of the coating material per 1 part by weight of the core substance.

### DETAILED DESCRIPTION OF THE INVENTION

The invention is described in more detail below.

As the specific examples of the core substance to be used in the invention, there are illustrated a powdery or granular flavor composition, colour material, acidity regulator, seasoning, sweetener, spice, vitamin, functional material or mixture thereof having an average particle size of 30 to 3000 µm. However, any substance that is solid at ordinary temperatures may be used. In cases when the core substance is a flavor composition, the invention provides a coated powder showing better flavor-keeping properties and better controlled flavor release in comparison with flavor powders produced by conventional coating methods, thus a flavor composition being one of preferred core substances.

Additionally, the flavor composition to be used in the invention as a core substance includes a flavor powder composition prepared by forming a flavor ingredient into a powdery form using an emulsifier, a carrier and the like, a flavor powder composition prepared by merely spraying a flavor against sugar or the like to adsorb thereon, and a flavor material which itself is solid at ordinary temperatures.

The flavor ingredient of the flavor composition to be used in the invention as a core substance includes any of conventionally known flavor ingredients. Such flavor ingredient is exemplified by citrous flavors such as orange, lemon and grapefruit; fruit type flavors such as apple, banana, grape, peach, strawberry and pineapple; mint type flavors such as peppermint and spearmint; spice type flavors such as pepper, cinnamon, nutmeg and clove; nut type flavors such as vanilla, coffee, cocoa and hazelnut; tea type flavors such as black tea and green tea; meat or marine product type flavors such as beaf, chicken, salmon and crab; and dairy type flavors such as milk and cheese. However, flavor ingredients to be used in the invention are not limited only to these.

Some of the above-described flavor ingredients may be used in the form of essential oil or oleoresin as well as mixed flavors. These flavors may be either natural flavors or synthetic flavors. Typical examples of the synthetic flavor include citral, geraniol, 1-menthol and vanillin. These flavors may be a mixture in any mixing ratio.

In addition, as the flavor composition, there may be used powder products of extracts of coffee, black tea or dried bonito obtained by supercritical fluid extraction, or natural fruit juices.

In using a colour material as the core substance material, every conventionally known colours may be used as the colour material. Specific examples of the colour material include oil-soluble natural colours such as β-carotene, paprika colour, annatto colour and chlorophyll and, further, natural colour materials such as turmeric colour, caramel colour, cochineal colour and grape skin extract. These colour materials are usually formed into powder using an emulsifier or a carrier to use as a core substance.

As the acidity regulator to be used as a core substance material, any of those which are generally used in foods may be used. Specific examples of such known acidity regulators include citric acid, malic acid, ascorbic acid, succinic acid and fumaric acid. These acidity regulators may be used as powders such as crystalline or granular powder composed of them, or powders prepared from them using a carrier. In addition, acetic acid formed into a powder form may also be used as the core substance.

As the sweetener to be used as a core substance material, any of those which have conventionally been used as sweeteners may be used. Typical examples of such sweeteners include sucrose, aspartame, paratinose, raffinose, trehalose, erythritol and xylitol. These are used as the core substance in the form of particles or granules of sweeteners or in a powdery form using a carrier with the sweetener. Of these sweeteners, those which are solid at ordinary temperatures are preferred.

As the spice to be used as a core substance material, any of those which have conventionally been used as spices may be used. Examples of the spices include powders of natural spices such as pepper, clove and mace and blends thereof. In addition, powders prepared from a spice oil or oleoresin prepared by extracting effective ingredients from these spices, using an emulsifier and a carrier, and a powder prepared by merely spraying the spice against sugar or the like to adsorb thereon are also preferred as the core substance.

As vitamins to be used as core substance materials, there are illustrated, for example, vitamin A, vitamin C, vitamin D, vitamin K and vitamin P.

As functional materials to be used as core substance materials, there are illustrated, for example, powdered form of polyphenols of perilla extract or buckwheat, propolis and royal jelly. As the functional materials, those which are solid at ordinary temperatures are preferred, but any one that can be formed into a powder with the aid of a carrier may also be used as the core substance.

As the fractionated yeast cell wall to be used in the invention as one component of the coating material, any of those which are described in Japanese Patent Laid-Open No. 44878/2000 may be used regardless of the kind of yeast, presence or absence of acid treatment, and presence or absence of a plasticizer. In using for foods or beverages, "Yeast Wrap" sold by Kirin Brewery Co., Ltd. is preferred in the point that the resultant coating material has extremely low oxygen permeability and moisture permeability.

Additionally, detailed descriptions on the method for obtaining the fractionated yeast cell wall to be used in the invention as one component of the coating material are omitted here since detailed descriptions are given in Japanese Patent Laid-Open No. 44878/2000, herein incorporated by reference. For example, Japanese Patent Laid-Open No. 44878/2000 describes in Example 1 that a beer yeast slurry obtained as a by-product from beer factories was subjected to centrifugal separation at 4500 rpm for 10 minutes, the resultant slurry of living yeast was suspended in water in a solid content of 5% by weight and, after allowing autolysis under the reaction conditions of 50°C for 17 hours, the suspension was again subjected to centrifugal separation to thereby obtain as fractionated yeast cell wall an autolysis residue from which soluble intracellular ingredients have been removed.

As the thckening polysaccharides to be used in the invention in combination with the fractionated yeast cell wall, there are illustrated carrageenan, carob bean gum and pullulan, with pullulan being preferred in the point of film-forming properties and heat resistance.

As the oligosaccharides, there are illustrated, for example, monosaccharides, disaccharides and trisaccharides, any of which may be used. Typical examples of the oligosaccharides include xylose, galactose, maltose, trehalose, paratinose and raffinose, with trehalose, paratinose and raffinose being preferred in the point of low hygroscopicity and physical and chemical stability of showing no reducing ability or markedly low reactivity.

As the hardened fats and oils, any of those fats hardened and oils which have a melting point of 40°C or higher than that may be used. As examples of hardened fats and oils to be preferably used in the invention, there are illustrated hardened fats and oils obtained by hydrogenation-treating food-grade liquid vegetable oils such as rape seed oil, soybean oil, cotton seed oil, safflower oil, sunflower oil, palm oil, coconut oil, olive oil, sesami oil, rice oil, corn oil and peanut oil, and hydrogenated products of beef tallow, lard and fish-and-whale oil.

As the waxes, solid esters composed of a higher fatty acid and a higher primary alcohol suffice, and examples of the waxes include rice bran wax, carnauba wax, whale wax and bees wax.

As the sugar alcohols, any of erythritol, mannitol and lactitol is used since they are acid-resistant and heat-resistant, do not colored by Maillard reaction and have excellent workability as a coating material. Erythritol, mannitol and lactitol are preferred due to their low hygroscopicity.

The starch hydrolyzate means dextrin, but hydrolysis of starch finally decomposes it to glucose. Degree of starch decomposition is represented by DE value (Dextrose Equivalent), which shows a percentage of direct reducing sugar in terms of glucose in the solids. Various properties of dextrin can be presumed using the DE value as an indication. In the invention, starch hydrolyzates of 20 to 50 in DE value are preferred. Kind of starting starch does not matter as long as the DE value of a particular starch hydrolyzate falls within this range.

The coating material is used in an amount of preferably 0.05 to 1.5 parts by weight per 1 part by weight of a core substance. If used in a less amount than this range, the coating material would fail to sufficiently coat the core substance such as a flavor powder whereas, if used in a more amount than is necessary, there would be obtained functionally unfavorable results when the resultant coated powder is added to, for example, foods or beveradges to impart flavor to final products. In the present invention, combined use of one or more members selected from among viscous polysaccharides, oligosaccharides, hardened fats and oils, waxes and erythritol, mannitol, lactitol and the starch hydrolyzate provides a coated powder more excellent in coating properties, oxidation stability, heat resistance, sustained and controlled flavor-releasing properties in comparison with the case of using a fractionated yeast cell wall not containing them as the coating material.

To illustrate the range of the amount of these materials to be used in combination with the fractionated yeast cell wall by reference to typical materials, the viscous polysaccharides such as pullulan are used in an amount of preferably 0.0001 to 5.0 parts by weight, more preferably 0.001 to 4.0 parts by weight based on 1 part by weight of the fractionated yeast cell wall. The oligosaccharides such as trehalose are used in an amount of preferably 0.01 to 5.0 parts by weight, more preferably 0.1 to 4.0 parts by weight, the sugar alcohols are used in an amount of preferably 0.0001 to 0.5 parts by weight, more preferably 0.001 to 0.3 parts by weight, and starch hydrolyzates such as cyclodextrin are used in an amount of preferably 0.0001 to 5.0 parts by weight, more preferably 0.001 to 4.0 parts by weight based on 1 part by weight of the fractionated yeast cell wall. If the amounts are less than the lower limits described above, effects by the combined use with the fractionated yeast cell wall might not be obtained. On the other hand, if more than the upper limits, no additional effects can be obtained with respect to the viscous polysaccharides, oilgosaccharides and starch hydrolyzates, thus such amounts exceeding the upper limits being economically useless and, with respect to materials classified as sugar alcohols, a step for drying them requires the upper limits.

In addition, the combined use of the fractionated yeast cell wall with at least one of the viscous polysaccharides and oligosaccharides provide a coated powder having a particularly enhanced oxidation stability. Thus, a coated powder having the functions of sufficient oxidation stability, heat resistance, controlled-release properties, and the like can be prepared by coating a core substance with a combination of a natural coating material composed of fractionated yeast cell wall and at least one member selected from the group consisting of the viscous polysaccharides, oligosaccharides, hardened fats and oils, waxes, erythritol, mannitol, lactitol and starch hydrolyzates.

As a method for coating a core substance with the coating material of the invention, any method can be employed that has so far been employed for coating a core substance with a coating material. For example, the coated powder of the invention can be produced by spraying the coating material of the invention to a stirred or fluidized core substance. Of the known coating methods, a method of conducting the coating while feeding the core substance in one direction in a large amount at a high speed with the aid of an up-current according to the Warster coating method in which a core substance is kept in a state of fluidized bed, and a coating material solution is sprayed upward from the bottom of the coating vessel is preferred. However, the coating method of the invention is not limited to this method. The coating material of the invention is sprayed against the fluidized bed usually in a state of being suspended in a mixed solvent composed of water or both water and an organic solvent. In using the hardened fats and oils or waxes, they are dissolved, the fractionated yeast cell wall is suspended therein, and the resulting suspension is sprayed against the fluidized bed. Therefore, as the coating apparatus, a fluidized-bed, granulating and drying coater is preferred. As such fluidized-bed, granulating and drying coater, there may be illustrated as a preferred one Multiplex model MP-01 manufactured by Powerex Co., Ltd. Drying temperature upon coating is properly decided depending upon kind of core substance, composition of the coating material, amount of coating, and kind of a solvent used. Drying is usually conducted at about 30°C with hardened fats and oils and waxes or at about 50 to 90°C with other materials. In addition, drying period is also properly decided depending upon drying temperature, kind of core substance, composition of the coating material, amount of coating and kind of a solvent used. Further, thickness of the coating material around the core substance is properly decided depending upon kind of the core substance and use of the coated powder.

The coated powder of the invention is used preferably in various foods and beverages. As examples of such foods and beverages, there are illustrated a wide variety of products of liquid beverages such as coffee, black tea, cocoa, alcoholic drinks, soft drinks and fruit drinks; various powdered beverages such as instant coffee; confectionery such as candy, chewing gum, tableted confections and chocolate; bakery products such as cookie and bread; Japanese confectionery; pastry such as cake; desserts such as yogurt and ice cream; dairy products such as cheese; meat and marine products such as ham, boiled fish paste and milled food; and cooked foods adapted for a microwave oven (frozen foods, retort foods).

For example, in cases when the coated powder is a powdered flavor, it is added to foods or beverages in an amount of desirably 0.05 to 5.0% by weight, preferably 0.5 to 3.0% by weight based on the foods or beverages, though the amount greatly changes depending upon kind of the powdered flavor or kind of food or beverage to which it is added. Addition is conducted in a known manner.

The coated powder obtained by the invention is also used for various perfumy products such as various cosmetics, aromatics and sanitary goods. To specifically illustrate these perfumy products, there are illustrated a wide variety of products: various cosmetics such as washing cosmetics such as soap, shampoo and hair rinse, cosmetics for hair such as hair dye and hair tonic; skin care cosmetics such as cream, lotion, eau de cologne and pack, makeup cosmetics such as face powder, foundation and cheek rouge, fragrant cosmetics such as perfume, suntan or sunscreen cosmetics, lip cosmetics such as lipstick and lip cream, mouth cosmetics such as tooth paste or powder and mouth wash, and bath cosmetics; aromatics such as deodorant and indoor aromatic; sanitary materials such as disinfectant and insecticide; and others such as bleaching agent, softener, detergent for food and washing detergent.

In adding the coated powder of powdered flavor to the perfumy products, it is added in an amount of preferably 0.05 to 30.0% by weight, though the amount greatly changes depending upon kind of the powdered flavor or kind of perfumy product to which it is added. Addition of the coated powder is conducted in a known manner.

The invention is described in more detail by reference to Examples which, however, do not limit the invention in any way.

### Example 1

80 g of menthol flavor was formed into a powder according to a known method (spray drying) using 320 g of gum Arabic (content of solids: 30%) as a carrier to prepare a menthol flavor-containing powder having an average particle size of 100 µm.

As a coating material, 2030g of a solution was prepared by adding 190 g (in terms of solids) of a fractionated yeast cell wall (Yeast Wrap; made by Kirin Brewery Co., Ltd.) and 10 g of pullulan to water. The above-described menthol flavor-containing powder was coated using this solution as a coating solution and using a coating machine, Multiplex model MP-01 (made by Powrex Co., Ltd.). The coating was conducted using 2030 g of the coating solution per 400 g of the core substance of menthol flavor-containing powder under the conditions of 70°C in feed gas temperature, 40°C in exhaust temperature and 4 g/min in coating solution-feeding rate, to thereby produce a menthol flavor-containing powdery product. Additionally, the amount of coating material used in Example 1 was 0.50 part by weight per 1 part by weight of the core substance.

### Examples 2 to 5

Menthol flavor-containing powdery products of Examples 2 to 5 were produced in the same manner as in Example 1 except for changing the amounts of fractionated yeast cell wall and pullulan as shown in Table 1. Additionally, the amount of coating material used in each Example was 0.50 parts by weight (Examples 2 to 4) or 2.0 part by weight (Example 5) per 1 part by weight of the core substance.

### Comparative Example 1

A menthol flavor-containing product (average particle size: 100 µm) was prepared in the same manner as in Example 1. A menthol flavor-containing powdery product coated with a coating material was produced in the same manner as in Example 1 using this product as a core substance except for using 12 g of the same fractionated yeast cell wall (Yeast Wrap; made by Kirin Brewery Co., Ltd.) and not using pullulan as the coating material.

### Comparative Example 2

A menthol flavor-containing powdery product of Comparative Example 2 was produced in the same manner as in Example 1 except for changing the amounts of the fractionated yeast cell wall and pullulan as shown in Table 2.

Formulations of the core substance and the coating materials used in Examples 1 to 5 are shown in Table 1, and that used in Comparative Examples 1 and 2 are shown in Table 2.

**Table 1**

| | | | Ex.1 | Ex.2 | Ex.3 | Ex.4 | Ex. 5 |
|---|---|---|---|---|---|---|---|
| Core Substance | Flavor | Menthol flavor | 80 | 80 | 80 | 80 | 80 |
| | Carrier | Gum Arabic (solid content: 30%) | 320 | 320 | 320 | 320 | 320 |
| Coating Material | | Fractionated yeast cell wall (a) | 190 | 180 | 80 | 40 | 720 |
| | | Pullulan (b) | 10 | 20 | 120 | 160 | 80 |
| | | (b) /( a) | 0.053 | 0.11 | 1.5 | 4.0 | 0.11 |

**Table 2**

| | | | Com. Ex.1 | Com. Ex.2 |
|---|---|---|---|---|
| Core Substance | Flavor | Menthol flavor | 80 | 80 |
| | Carrier | Gum arabic (solid content: 30%) | 320 | 320 |
| Coating Material | | Fractionated yeast cell wall (a) | 12 | 200 |
| | | Pullulan (b) | - | - |
| | | (b) /( a) | - | - |

### Example 6

### (Functional evaluation test using chewing gum)

The menthol flavor-containing, coated powdery product obtained in Example 1 was added to a chewing gum matrix composed of 20 parts by weight of gum base, 66 parts by weight of powder sugar and 14 parts by weight of corn syrup in such amount that 1.0% of the flavor ingredient was incorporated (i.e., 1.0% of menthol was incorporated based on the chewing gum matrix). This flavored chewing gum matrix was kneaded at about 40°C for 10 minutes, spread, and formed into 3 g sheet gums. The thus obtained sheet gums were taken as samples 1 of the invention and were subjected to the following functional test by special panels. Results thus obtained are shown in Table 3.

### (Evaluation test by special panels)

Functional test was conducted by 5 special panels. Evaluation was conducted in terms of flavor-lasting properties.

The sample chewing gums were chewed for 5 minutes, and flavor given off was compared with time, evaluation being conducted according to the following 4 grades. Evaluation results were shown in terms of number of panels for respective grades.

### (Standard for functional evaluation of chewing gum)

A: Flavor did not last, and poorly balanced with dissolution of sugar component of the chewing gum.
B: Flavor was given off at a common level.
C: Although lasted sufficiently, flavor was poorly balanced with dissolution of sugar component of the chewing gum.
D:Flavor lasted excellently, and well balanced with dissolution of sugar component of the chewing gum.

### Examples 7 to 10

Sheet gums were produced and subjected to the evaluation test by special panels in the same manner as in Example 6 except for using each of the menthol flavor-containing, coated powdry products obtained in Examples 2 to 5 in place of the menthol flavor-containing, coated powdery product obtained in Example 1 to prepare samples 2 to 5 in accordance with the invention. Results thus obtained are shown in Table 3.

### Comparative Examples 3 and 4

Sheet gums were produced and subjected to the evaluation test by special panels in the same manner as in Example 6 except for using each of the menthol flavor-containing, coated powdry products obtained in Comparative Examples 1 and 2 in place of the menthol flavor-containing, coated powdery product obtained in Example 1 to prepare comparative samples 1 and 2. Results thus obtained are shown in Table 3.

**Table 3**

| Functional Evaluation | A | B | C | D |
|---|---|---|---|---|
| Sample of the invention 1 | 0 | 1 | 0 | 4 |
| Sample of the invention 2 | 0 | 3 | 0 | 2 |
| Sample of the invention 3 | 0 | 2 | 0 | 3 |
| Sample of the invention 4 | 0 | 1 | 0 | 4 |
| Sample of the invention 5 | 0 | 0 | 5 | 0 |
| Comparative sample 1 | 3 | 2 | 0 | 0 |
| Comparative sample 2 | 4 | 1 | 0 | 0 |

As is apparent from Table 3, samples of the invention all showed excellent flavor-lasting properties, whereas comparative samples 1 and 2 not using pullulan showed poor flavor-lasting properties, and comparative sample 2 containing an increased amount of Yeast rap poorly gave off flavor, lacked balance with dissolution time of the sugar component of the chewing gum and lacked delicious taste. Additionally, even when the fractionated yeast cell wall was used in combination with pullulan, use of an exess amount of the coating material as in the sample 5 of the invention resulted in insufficient balance with the period of dissolution of the sugar component, though flavor-lasting properties were obtained.

### Example 11

A lemon flavor-containing, coated powdery product having an average particle size of 80 µm was produced in the same manner as in Example 1 except for using lemon flavor in place of menthol flavor and using paratinose in place of pullulan. Additionally, formulations of the core substance and the coating material are shown in Table 4. The amount of used coating material was 0.50 part by weight per 1 part by weight of the core substance. The thus obtained lemon flavor-containing, coated powdered product was referred to as sample 6 of the invention and was subjected to abuse text according to the following evaluation test. Results of the evaluation are shown in terms of number of panels for respective grades.

### (Functional evaluation test on lemon flavor)

The lemon flavor-containing, coated powdered product was subjected to abuse text at 60°C for 5 weeks. Functional evaluation was conducted by 5 special panels using a 0.1% aqueous solution of the coated powder, with rating degree of deterioration of lemon-like flavor in the following three levels.

### (Standard of functional evaluation of lemon-like flavor)

A: Lemon-like flavor was deteriorated.
B: Lemon-like flavor was given off at an ordinary level.
C: Lemon-like flavor was retained at an excellent level.

### Comparative Example 5

A lemon flavor-containing, coated powdery product was produced in the same manner as in Example 1 except for not using pullulan in the coating material and using 12 g of the same fractionated yeast cell wall (made by Kirin Beer Co., Ltd.; Yeast rap) as in Example 11. Additionally, formulations of the core substance and the coating material are shown in Table 4. The thus obtained lemon flavor-containing, coated powdery product was referred to as comparative sample 3, and was subjected to the same deterioration test as in Example 11. Results thus obtained are shown in Table 5.

**Table 4**

| | | | Ex.11 | Com. Ex.5 |
|---|---|---|---|---|
| Core Substance | Flavor | Lemon flavor | 80 | 80 |
| | Carrier | Gum arabic (solid content: 30%) | 320 | 320 |
| Coating Material | | Fractionated yeast cell wall (a) | 190 | 12 |
| | | Paratinose (c) | 10 | - |
| | | (c) / (a) | 0.053 | - |

**Table 5**

| Functional Evaluation | A | B | C |
|---|---|---|---|
| Sample 6 of the invention | 0 | 0 | 5 |
| Comparative sample 3 | 5 | 0 | 0 |

As is apparent from Table 5, comparative sample 3 not using paratinose in combination underwent deterioration of lemon-like flavor, whereas sample 6 of the invention using paratinose in combination showed markedly excellent lemon-like flavor-lasting properties.

### Example 12

A butter flavor-containing coated powdery product (average particle size: 120 µm) was produced in the same manner as in Example 1 except for using butter flavor in place of menthol flavor. Additionally, formulations of the core substance and the coating material are shown in Table 6. The amount of coating material used in Example 12 was 0.50 part by weight per 1 part by weight of the core substance.

### Example 13

### (Functional evaluation test using baked confection (cookie) Formulation of cookie matrix

| | |
|---|---|
| Weak flour | 48 parts by weight |
| Shortening | 30 parts by weight |
| Water | 20 parts by weight |
| Sugar | 1 part by weight |
| Salt | 1 part by weight |

According to the above formulation, shortening was first placed as a fat-and-oil component in a mixer, and was stirred at a rate of 60 to 80 rpm to thereby introduce and distribute air therein, followed by adding thereto sugar and stirring. Then, water was added thereto, and the mixture was mixed so as to make the whole mixture uniform, followed by mixing with weak flour and salt to prepare a cookie dough. This dough was separated into 20 g portions, and the butter flavor-containing coated powdery product obtained in Example 12 was added to each of the portions so that the content of the flavor became 0.2% based on the dough, followed by sufficient stirring. After molding each portion, molded portions were baked in a 150°C oven for 20 minutes to produce cookies. The cookies were referred to as sample 7 of the invention and were subjected to the following functional test by special panels. Results thus obtained are shown in Table 7. Results of the evaluation was shown in terms of number of the panels for respective grades.

### (Evaluation test by special panels)

Functional test was conducted by 5 special panels. Evaluation was conducted in terms of flavor-lasting properties.

The sample cookies were bit for 5 minutes in the mouth, and butter flavor given off upon swallowing down and flavor-lasting properties were compared, evaluation being conducted according to the following 3 grades.

### (Standard for functional evaluation of butter flavor)

A: Flavor did not last at all.
B: Flavor was given off at a common level.
C: Flavor lasted at an extremely excellent level.

### Comparative Example 6

A butter flavor-containing coated powdery product (average particle size: 120 µm) was produced in the same manner as in Example 12 except for not using pullulan in the coating material and using 12 g of fractionated yeast cell wall (Kirin Brewery Co., Ltd.; Yeast Wrap) as the coating material. Additionally, formulations of the core substance and the coating material are shown in Table 6.

### Comparative Example 7

Cookies were produced and evaluated by special panels in the same manner as in Example 13 except for using the butter flavor-containing powdery product prepared in Comparative Example 6 as butter flavor-containing powdery product and referring the thus obtained cookies as comparative sample 4. Results thus obtained are shown in Table 7.

**Table 6**

| | | | Ex. 12 | Com. Ex. 6 |
|---|---|---|---|---|
| Core Substance | Flavor | Butter flavor | 80 | 80 |
| | Carrier | Gum arabic (solid content: 30%) | 320 | 320 |
| Coating Material | | Fractionated yeast cell wall (a) | 190 | 12 |
| | | Pullulan (b) | 10 | - |
| | | (b) /(a) | 0.053 | - |

**Table 7**

| Functional Evaluation | A | B | C |
|---|---|---|---|
| Sample 7 of the invention | 0 | 1 | 4 |
| Comparative sample 4 | 3 | 2 | 0 |

As is apparent from Table 7, comparative sample 4 not using pullulan showed deteriorated flavor-lasting properties in comparison with sample 7 of the invention using pullulan.

### Example 14

80 g of grapefruit flavor was formed into a powder according to spray drying method using 320 g of gum arabic (content of solids: 30%) as a carrier to prepare a grapefruit flavor-containing powder having an average particle size of 150 µm.

As a coating material, 2030g of a solution was prepared by adding 96 g (in terms of solids) of a fractionated yeast cell wall (Yeast Wrap; made by Kirin Brewery Co., Ltd.) and 24 g of trehalose to water. The above-described grapefruit flavor-containing powder was coated using this solution as a coating solution and using a coating machine, Multiplex model MP-01 (made by Powrex Co., Ltd.). The coating was conducted using 2030 g of the coating solution per 400 g of the core substance of grapefruit flavor-containing powder under the conditions of 70°C in feed gas temperature, 40°C in exhaust temperature and 4 g/min in coating solution-feeding rate, to thereby produce a grapefruit flavor-containing powdery product. Additionally, formulations of the core substance and the coating material are shown in Table 8. The amount of coating material used was 0.30 part by weight per 1 part by weight of the core substance.

The thus obtained grapefruit flavor-containing, coated powdery product was referred to as sample 8 of the invention and was subjected to abuse test according to the following functional evaluation test. Results of the evaluation are shown in Table 9. The evaluation results are shown in terms of number of panels for respective scores.

### (Functional evaluation test on grapefruit flavor)

The grapefruit flavor-containing, coated powdered product was subjected to abuse text at 60°C for 5 weeks. Functional evaluation was conducted by 5 special panels using a 0.1% aqueous solution of the coated powder, with rating degree of deterioration of grapefruit-like flavor in the following three levels.

### (Standard of functional evaluation of grapefruit-like flavor)

A: Grapefruit-like flavor was deteriorated.
B: Grapefruit-like flavor was given off at an ordinary level.
C: Grapefruit-like flavor was retained at an excellent level.

### Example 15

A grapefruit flavor-containing, coated powdery product was produced in the same manner as in Example 14 except for using cyclodextrin in place of trehalose to obtain a grapefruit flavor-containing powdery product (average particle size: 150 µm). Additionally, formulations of the core substance and the coating material are shown in Table 8.

The thus obtained grapefruit flavor-containing, coated powdery product was referred to as sample 9 of the invention, and was subjected to the same deterioration test as in Example 14. Results thus obtained are shown in Table 9.

### Comparative Example 8

A grapefruit flavor-containing powdery product was produced in the same manner as in Example 14. A grapefruit flavor-containing coated powdery product (average particle size: 150 µm) was produced in the same manner as in Example 14 using this powdery product except for not using trehalose and using 120 g of the same fractionated yeast cell wall (Kirin Brewery Co., Ltd.; Yeast Wrap) as in Example 14. Additionally, formulations of the core substance and the coating material are shown in Table 8.

The thus obtained grapefruit flavor-containing, coated powdery product was referred to as comparative sample 5, and was subjected to the same deterioration test as in Example 14. Results thus obtained are shown in Table 9.

### Example 16

### (Functional evaluation test using baked confection (cookie)

Cookies were produced in the same manner as in Example 13 except for using the grapefruit flavor-containing powdery product obtained in Example 14 in place of the butter flavor-containing powdery product. The cookies were referred to as sample 10 of the invention and were subjected to the evaluation test by special panels as in Example 13. Results thus obtained are shown in Table 10.

### Example 17 and Comparative Example 9

Cookies were produced in the same manner as in Example 13 except for using the grapefruit flavor-containing powdery products produced in Example 15 and Comparative Example 8, respectively, as the grapefruit flavor-containing powdery product. The thus obtained cookies were referred to as sample 11 of the invention and comparative sample 6 and were subjected to the evaluation test by special panels as in Example 13. Results thus obtained are shown in Table 10.

### (Standard of functional evaluation of grapefruit-like flavor)

A: Grapefruit-like flavor did not remain at all.
B: Grapefruit-like flavor remained at an ordinary level.
C: Grapefruit-like flavor remained at an excellent level.

**Table 8**

| | | | Ex.14 | Ex.15 | Com. Ex. 8 |
|---|---|---|---|---|---|
| Core Substance | Flavor | Grapefruit flavor | 80 | 80 | 80 |
| | Carrier | Gum arabic (solid content: 30%) | 320 | 320 | 320 |
| Coating Material | | Fractionated yeast cell wall (a) | 96 | 96 | 120 |
| | | Trehalose (d) | 24 | - | - |
| | | Cyclodextrin(e) | - | 24 | - |
| | | (d) / (a) or (e) / (a) | 0.25 | 0.25 | - |

**Table 9**

| Functional Evaluation | A | B | C |
|---|---|---|---|
| Sample 8 of the invention | 0 | 0 | 5 |
| Sample 9 of the invention | 0 | 2 | 3 |
| Comparative sample 5 | 5 | 0 | 0 |

**Table 10**

| Functional Evaluation | A | B | C |
|---|---|---|---|
| Sample 10 of the invention | 0 | 1 | 4 |
| Sample 11 of the invention | 0 | 2 | 3 |
| Comparative sample 6 | 0 | 4 | 1 |

It is apparent from Tables 9 and 10 that flavor-containing powders showing excellent flavor-maintaining properties and flavor-lasting properties by using as a coating material a combination of trehalose or cyclodextrin and the fractionated yeast cell wall.

### Example 18

120 g of citric acid as an acidity regulator was formed into a powder according to spray drying method using 280 g of dextrin (content of solids: 95%) as a carrier to prepare a powder having an average particle size of 50 µm.

As a coating material, 2030 g of a solution was prepared by adding 180 g (in terms of solids) of a fractionated yeast cell wall (Yeast Wrap; made by Kirin Brewery Co., Ltd.) and 20 g of pullulan to water. The above-described acidity regulator-containing powder was coated using this solution as a coating solution and using a coating machine, Multiplex model MP-01 (made by Powrex Co., Ltd.). The coating was conducted using 2030 g of the coating solution per 400 g of the core substance of acidity regulator-containing powder under the conditions of 70°C in feed gas temperature, 40°C in exhaust temperature and 4 g/min in coating solution-feeding rate, to thereby produce an acidity regulator-containing powdery product. Additionally, formulations of the core substance and the coating material are shown in Table 11. The amount of coating material used was 0.50 part by weight per 1 part by weight of the core substance.

### Example 19

A spice-containing, coated powdery product was produced in the same manner as in Example 18 except for using 400 g of black pepper powder having an average particle size of 250 µm as a core substance in place of the acidity regulator-containing powder. Additionally, formulations of the core substance and the coating material are shown in Table 11. The amount of coating material used was 0.50 part by weight per 1 part by weight of the core substance.

### Example 20

A vitamin C-containing, coated powdery product was produced in the same manner as in Example 18 except for using 400 g of vitamin C powder having an average particle size of 40 µm as a core substance in place of the acidity regulator-containing powder. Additionally, formulations of the core substance and the coating material are shown in Table 11. The amount of coating material used was 0.50 part by weight per 1 part by weight of the core substance.

**Table 11**

| | | | Ex. 18 | Ex. 19 | Ex. 20 |
|---|---|---|---|---|---|
| Core Substance | Acidity regulator | Citric acid | 120 | - | - |
| | Spice | Black pepper powder | - | 400 | - |
| | Vitamin | Vitamin C powder | - | - | 400 |
| | Carrier | Dextrin (solid content:95%) | 280 | - | - |
| Coating Material | | Fractionated yeast cell wall (a) | 180 | 180 | 180 |
| | | Pullulan (b) | 20 | 20 | 20 |
| | | (b) / (a) | 0.11 | 0.11 | 0.11 |

### Example 21

180 g of dextrin and 100 g of lactose, which were both carriers, were mixed and gradually added dropwise to 120 g of liquid cheese flavor, followed by further stirring. After uniformly mixing the flavor, the mixture was sieved to prepare a cheese flavor powder having an average particle size of 500 µm.

As a coating material, 2030g of a solution was prepared by adding 96 g (in terms of solids) of a fractionated yeast cell wall (Yeast wrap; made by Kirin Brewery Co., Ltd.) and 24 g of trehalose to water. The above-described cheese flavor-containing powder was coated using this solution as a coating solution and using a coating machine, Multiplex model MP-01 (made by Powrex Co., Ltd.). The coating was conducted using 2030 g of the coating solution per 400 g of the core substance of acidity regulator-containing powder under the conditions of 70°C in feed gas temperature, 40°C in exhaust temperature and 4 g/min in coating solution-feeding rate, to thereby produce a cheese flavor-containing powdery product. Additionally, formulations of the core substance and the coating material are shown in Table 12. The amount of coating material used was 0.3 part by weight per 1 part by weight of the core substance.

**Table 12**

| | | | |
|---|---|---|---|
| Core Substance | Flavor | Cheese flavor | 120 |
| | Carrier | Lactose | 100 |
| | | Dextrin | 180 |
| Coating Material | | Fractionated yeast cell wall (a) | 96 |
| | | Trehalose (d) | 24 |
| | | (d) / (a) | 0.25 |

### Example 22

200 g of fine granulated sugar and 164 g of dextrin, which were both carriers, were mixed under heating to obtain a molten product. To this was added 36 g of orange flavor and, when the mixture became uniform, it was added to anextruder having an extrusion plate. After extrusion, the extruded product was dried to prepare an orange extrusion flavor having an average particle size of 1000 µm.

As a coating material, 2030 g of a solution was prepared by adding 180 g (in terms of solids) of a fractionated yeast cell wall (Yeast wrap; made by Kirin Brewery Co., Ltd.) and 20 g of pullulan to water. The above-described orange flavor-containing powder was coated using this solution as a coating solution and using a coating machine, Multiplex model MP-01 (made by Powrex Co., Ltd.). The coating was conducted using 2030 g of the coating solution per 400 g of the core substance of orange extrusion flavor under the conditions of 70°C in feed gas temperature, 40°C in exhaust temperature and 4 g/min in coating solution-feeding rate, to thereby produce a coated orange extrusion flavor. Additionally, formulations of the core substance and the coating material are shown in Table 13. The amount of coating material used was 0.50 part by weight per 1 part by weight of the core substance.

**Table 13**

| | | | |
|---|---|---|---|
| Core Substance | Flavor | Orange flavor | 36 |
| | Carrier | Fine granulated sugar | 200 |
| | | Dextrin | 164 |
| Coating Material | | Fractionated yeast cell wall (a) | 180 |
| | | Pullulan (b) | 20 |
| | | (b) / (a) | 0.11 |

As has been described in detail hereinbefore, the invention provides a coated powder comprising a core substance of a flavor-containing composition which, when used to impart flavor to foods, beverages or perfumy cosmetics, does not undergo deterioration or disappearance of flavor in spite of heat treatment, which gives off flavor in a controlled manner of gradual and lasting release within the mouth upon uptake, and which shows an excellent oxidation stability. In addition, when other substances than the flavor composition are used as the core substance, the coating material of the invention provides good coating properties, and the resultant coated powder shows excellent hygroscopicity resistance, oxidation stability and heat resistance.

While the invention has been described in detail and with reference to specific embodiments thereof, it will be apparent to one skilled in the art that various changes and modifications can be made therein without departing from the spirit and scope thereof.

This application is based on Japanese patent applications No. 2000-159675 filed on May 30, 2000 and No. 2001-92589 filed on March 28, 2001, the entire contents thereof being hereby incorporated by reference.

## Claims

1. A coating material comprising a fractionated yeast cell wall and at least one member selected from the group consisting of viscous polysaccharides, oligosaccharides, hardened fats and oils, waxes, erythritol, mannitol, lactitol and starch hydrolyzates.

2. The coating material as described in claim 1, wherein the viscous polysaccharide is pullulan.

3. The coating material as described in claim 1 or 2, wherein the oligosaccharide is at least one member selected from the group consisting of trehalose, paratinose and raffinose.

4. A coated powder comprising a core substance in a powdery or granular form having an average particle size of 30 to 3000 µm, one part by weight of said core substance being coated with 0.05 to 1.5 parts by weight of a coating material according to any one of claims 1 to 3.

5. The coated powder as described in claim 4, wherein the viscous polysaccharide is pullulan.

6. The coated powder as described in claim 4 or 5 wherein the oligosaccharide is at least one member selected from the group consisting of trehalose, palatinose and raffinose.

7. The coated powder as described in one of claims 4 to 6, wherein the core substance is a flavor composition, a colour material, an acidity regulator, a seasoning, a sweetener, a spice, a vitamin, a functional material or a mixture of two or more of them.

8. The coated powder as described in claim 7, wherein the core substance is a flavor composition.

9. A food or beverage composition which comprises the coated powder described in one of claims 4 to 8.

10. A perfumery cosmetic composition which comprises the coated powder described in one of claims 4 to 8.

11. A process for producing a coated powder according to any one of claims 4 to 8, which comprises spraying a solution of a coating material comprising a fractionated yeast cell wall and at least one member selected from the group consisting of viscous polysaccharides, oligosaccharides, hardened fats and oils, waxes, erythritol, mannitol, lactitol and starch hydrolyzates, against a core substance in a powdery or granular form of 30 to 3000 µm in an average particle size under stirring or in a fluidized state to thereby coat the core substance with 0.05 to 1.5 parts by weight of the coating material per 1 part by weight of said core substance.

## Patentansprüche

1. Beschichtungsmaterial, umfassend eine fraktionierte Hefezellwand und wenigstens einen Bestandteil, ausgewählt aus der Gruppe bestehend aus viskosen Polysacchariden, Oligosacchariden, gehärteten Fetten und Ölen, Wachsen, Erythritol, Mannitol, Lactitol und Stärkehydrolysaten.

2. Beschichtungsmaterial nach Anspruch 1, wobei das viskose Polysaccharid Pullulan ist.

3. Beschichtungsmaterial nach Anspruch 1 oder 2, wobei das Oligosaccharid wenigstens ein Bestandteil, ausgewählt aus der Gruppe bestehend aus Trehalose, Palatinose und Raffinose ist.

4. Beschichtetes Pulver, umfassend eine Kernsubstanz in pulverförmiger oder granulärer Form mit einer mittleren Teilchengröße von 30 bis 3000 µm, wobei ein Gewichtsteil der Kernsubstanz mit 0,05 bis 1,5 Gewichtsteilen eines Beschichtungsmaterials nach einem der Ansprüche 1 bis 3 beschichtet ist.

5. Beschichtetes Pulver nach Anspruch 4, wobei das viskose Polysaccharid Pullulan ist.

6. Beschichtetes Pulver nach Anspruch 4 oder 5, wobei das Oligosaccharid wenigstens ein Bestandteil, ausgewählt aus der Gruppe bestehend aus Trehalose, Palatinose und Raffinose ist.

7. Beschichtetes Pulver nach einem der Ansprüche 4 bis 6, wobei die Kernsubstanz eine Aromastoffzusammensetzung, ein Farbmaterial, ein Säureregulator, eine Würze, ein Süßungsmittel, ein Gewürz, ein Vitamin, ein funktionelles Material oder ein Gemisch aus zwei oder mehr von ihnen ist.

8. Beschichtetes Pulver nach Anspruch 7, wobei die Kernsubstanz eine Aromastoffzusammensetzung ist.

9. Lebensmittel- oder Getränkezusammensetzung, welche das beschichtete Pulver nach einem der Ansprüche 4 bis 8 umfasst.

10. Parfümeriekosmetikzusammensetzung, welche das beschichtete Pulver nach einem der Ansprüche 4 bis 8 umfasst.

11. Verfahren zum Herstellen eines beschichteten Pulvers nach einem der Ansprüche 4 bis 8, welches das Sprühen einer Lösung eines Beschichtungsmaterials, umfassend eine fraktionierte Hefezellwand und wenigstens einen Bestandteil, ausgewählt aus der Gruppe bestehend aus viskosen Polysacchariden, Oligosacchariden, gehärteten Fetten und Ölen, Wachsen, Erythritol, Mannitol, Lactitol und Stärkehydrolysaten, gegen eine Kernsubstanz in pulverförmiger oder granulärer Form mit einer mittleren Teilchengröße von 30 bis 3000 µm unter Rühren oder in einem fluidisierten Zustand umfasst, um dadurch die Kernsubstanz mit 0,05 bis 1,5 Gewichtsteilen des Beschichtungsmaterials pro 1 Gewichtsteil der Kernsubstanz zu beschichten.

## Revendications

1. Matériau d'enrobage comprenant une paroi de cellule de levure fractionnée et au moins un membre choisi dans l'ensemble comprenant les polysaccharides visqueux, les oligosaccharides, les graisses durcies et les huiles, les cires, l'érythritol, le mannitol, le lactitol et les hydrolysats d'amidon.

2. Matériau d'enrobage selon la revendication 1, dans lequel le polysaccharide visqueux est le pullulane.

3. Matériau d'enrobage selon la revendication 1 ou 2, dans lequel le polysaccharide est au moins un membre choisi dans l'ensemble comprenant le tréhalose, le paratinose et le raffinose.

4. Poudre enrobée comprenant une substance de coeur sous forme pulvérulent ou granulaire ayant une granulométrie moyenne de 30 à 3000 µm, une partie en poids de ladite substance de coeur étant enrobée de 0,05 à 1,5 parties en poids d'un matériau d'enrobage selon l'une quelconque des revendications 1 à 3.

5. Poudre enrobée selon la revendication 4, dans laquelle le polysaccharide visqueux est le pullulane.

6. Poudre enrobée selon la revendication 4 ou 5, dans laquelle l'oligosaccharide est au moins un membre choisi dans l'ensemble comprenant le tréhalose, le palatinose et le raffinose.

7. Poudre enrobée selon l'une des revendications 4 à 6, dans laquelle la substance de coeur est une composition aromatisante, une matière colorante, un régulateur d'acidité, un assaisonnement, un édulcorant, une épice, une vitamine, un matériau fonctionnel ou un mélange d'au moins deux d'entre eux.

8. Poudre enrobée selon la revendication 7, dans laquelle la substance de coeur est une substance aromatisante.

9. Composition d'aliment ou de boisson, qui comprend la poudre enrobée selon l'une des revendications 4 à 8.

10. Composition cosmétique de parfumerie, qui comprend la poudre enrobée selon l'une des revendications 4 à 8.

11. Procédé de production d'une poudre enrobée selon l'une quelconque des revendications 4 à 8, qui comprend la pulvérisation d'une solution d'un matériau d'enrobage comprenant une paroi de cellule de levure fractionnée et au moins un membre choisi dans l'ensemble comprenant les polysaccharides visqueux, les oligosaccharides, les graisses durcies et les huiles, les cires, l'érythritol, le mannitol, le lactitol et les hydrolysats d'amidon, contre une substance de coeur sous forme pulvérulente ou granulaire ayant une granulométrie moyenne de 30 à 3000 µm, sous agitation ou à l'état fluidisé, pour de ce fait enrober une partie en poids de ladite substance de coeur de 0,05 à 1,5 parties en poids du matériau d'enrobage.
